# EUROPEAN PATENT APPLICATION

(11) **EP 4 115 830 A1**
(43) Date of publication of application: **11.01.2023**
(21) Application number: 20964380.8
(22) Date of filing: 10.12.2020
(51) Int. Cl.: A61B 18/14, A61N 1/36, A61N 1/05, A61B 18/00

(54) **ELECTRODE DEVICE**

(30) Priority: 03.12.2020 KR 20200167806
(71) Applicant: Deepqure Inc., Seoul 03136 (KR)
(72) Inventor: JEONG, Chang Wook, Seoul 05812 (KR); CHANG, Seok Joo, Seoul 08809 (KR); JO, Seok Hyeon, Namyangju-si Gyeonggi-do 12167 (KR); BACH, Du Jin, Seongnam-si Gyeonggi-do 13506 (KR)
(74) Representative: Manitz Finsterwald Patent- und Rechtsanwaltspartnerschaft mbB
(86) International application number: PCT/KR2020/018057
(87) International publication number: WO 2022/119032

(57) **Abstract**

An electrode apparatus for nerve denervation or modulation in body includes a main body including a shaft; an electrode unit formed to be drawn out from one end of the shaft and configured to denervate or modulate at least some of nerves on a tube in the body; and an electrode guide deformed into a wound state to bring the electrode unit into contact with the tube in the body. The electrode guide includes a body portion which is spaced apart from the electrode unit to enclose the circumference of the tube in the wound state; and a coupling portion which is connected to the end of the body portion and to which an end of the electrode unit is fixed.

## Description

### TECHNICAL FIELD

The present disclosure relates to an electrode apparatus for nerve denervation or modulation in body.

### BACKGROUND

A denervation is a surgical procedure intended to control an abnormally overactive autonomic nervous system by damaging specific nerves. For example, a renal denervation can treat hypertension and heart diseases by damaging renal sympathetic nerves directed to the kidney, and a pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves. For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode. Also, it is necessary to suppress or minimize unnecessary energy transfer and contact in the body.

### DISCLOSURE OF THE INVENTION

### PROBLEMS TO BE SOLVED BY THE INVENTION

The present disclosure is conceived to provide an electrode apparatus including an electrode component to be in contact with a human body and an electrode guide coupled to the electrode component in minimum contact with each other and configured to manipulate the electrode component.

The problems to be solved by the present disclosure are not limited to the above-described problems. There may be other problems to be solved by the present disclosure.

### MEANS FOR SOLVING THE PROBLEMS

According to an aspect of the present disclosure, an electrode apparatus for nerve denervation or modulation in body includes a main body including a shaft; an electrode unit formed to be drawn out from one end of the shaft and configured to denervate or modulate at least some of nerves on a tube in the body; and an electrode guide deformed into a wound state to bring the electrode unit into contact with the tube in the body. The electrode guide includes a body portion which is spaced apart from the electrode unit to enclose the circumference of the tube in the wound state; and a coupling portion which is connected to the end of the body portion and to which an end of the electrode unit is fixed.

According to the present disclosure, the coupling portion includes a first clamping piece fixed to the body portion; and a second clamping piece coupled to the first clamping piece with a portion of the electrode unit interposed therebetween.

According to the present disclosure, any one of the first clamping piece and the second clamping piece includes a protrusion protruding in one direction, and the other one of the first clamping piece and the second clamping piece includes a fastening groove recessed corresponding to the protrusion. A through-hole through which the protrusion penetrates is formed at a portion of the electrode unit.

According to the present disclosure, the electrode unit is extended to enclose the second clamping piece and inserted between the first clamping piece and the second clamping piece.

According to the present disclosure, the electrode guide further includes a joint wire which penetrates the body portion and is coupled to the second clamping piece to guide the body portion to be in the wound state. The second clamping piece includes a wire groove formed to allow insertion of an end of the joint wire; and a fixing plate configured to close a portion of the wire groove in order to suppress deviation of the end of the joint wire from the wire groove.

According to the present disclosure, the electrode unit includes a base layer; an electrode layer disposed on the base layer; and a top layer disposed to overlap a portion of the electrode layer with the electrode layer interposed therebetween.

According to the present disclosure, the coupling portion includes a first clamping piece fixed to the body portion; and a second clamping piece coupled to the first clamping piece with a portion of at least one of the base layer and the top layer interposed therebetween.

According to the present disclosure, any one of the first clamping piece and the second clamping piece includes a protrusion protruding in one direction, and the other one of the first clamping piece and the second clamping piece includes a fastening groove recessed corresponding to the protrusion. At least one of the base layer and the top layer includes a through-hole through which the protrusion penetrates.

According to the present disclosure, at least one of the base layer and the top layer is extended to enclose a part of the coupling portion and inserted and fixed in the coupling portion.

According to the present disclosure, the electrode unit further includes a reinforcing layer of which a part is coupled to the base layer and the other part is inserted and fixed in the coupling portion.

According to the present disclosure, at least one of the base layer and the top layer is extended to enclose a part of the coupling portion and is inserted and fixed in the coupling portion in an opposite direction to a direction of insertion of the other part of the coupling portion.

According to the present disclosure, the electrode unit further includes a sensor unit disposed on the base layer and configured to sense a temperature.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the above-described aspects of the present disclosure, an electrode guide and an electrode unit can be spaced apart from each other in most sections in a wound state. Therefore, in a state where the separation space between the electrode unit and the electrode guide is exposed to air, a surgical procedure can be performed and heat generated from the electrode unit can be easily dissipated through convective heat transfer.

Also, according to any one of the above-described aspects of the present disclosure, while a coupling (assembly) point between the electrode unit and the electrode guide is minimized, the electrode unit can be stably supported by a coupling portion of the electrode guide. Thus, durability and reliability can be secured. Further, the electrode unit and the electrode guide can be easily assembled with each other.

Furthermore, since the electrode guide deformed into the wound state by power and the electrode unit in close contact with a tube in the body are configured separately from each other, it is possible to suppress the effects of power transmission, for example, transmission of a vibration or force to the tube in the body.

### BRIEF DESCRIPTION OF THE DRAWINGS

**FIG. 1** is a side view of an electrode apparatus according to an embodiment of the present disclosure.
**FIG. 2** is a perspective view illustrating a wound state of an electrode guide illustrated in FIG. 1.
**FIG. 3A** through **FIG. 3C** illustrate a process of deforming the electrode guide into a wound state according to an embodiment of the present disclosure.
**FIG. 4** is a perspective view illustrating a body portion and a coupling portion illustrated in **FIG. 2****.**
**FIG. 5** is an exploded perspective view illustrating an electrode unit of the coupling portion illustrated in **FIG. 4****.**
**FIG. 6** is a perspective view illustrating a state in which the electrode unit and the coupling portion are coupled to each other illustrated in **FIG. 5****.**
**FIG. 7** is a plan view illustrating a portion of the electrode unit illustrated in **FIG. 5****.**

### BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Hereinafter, an exemplary embodiment of the present disclosure will be described in detail with reference to the accompanying configuration views or process flowcharts.

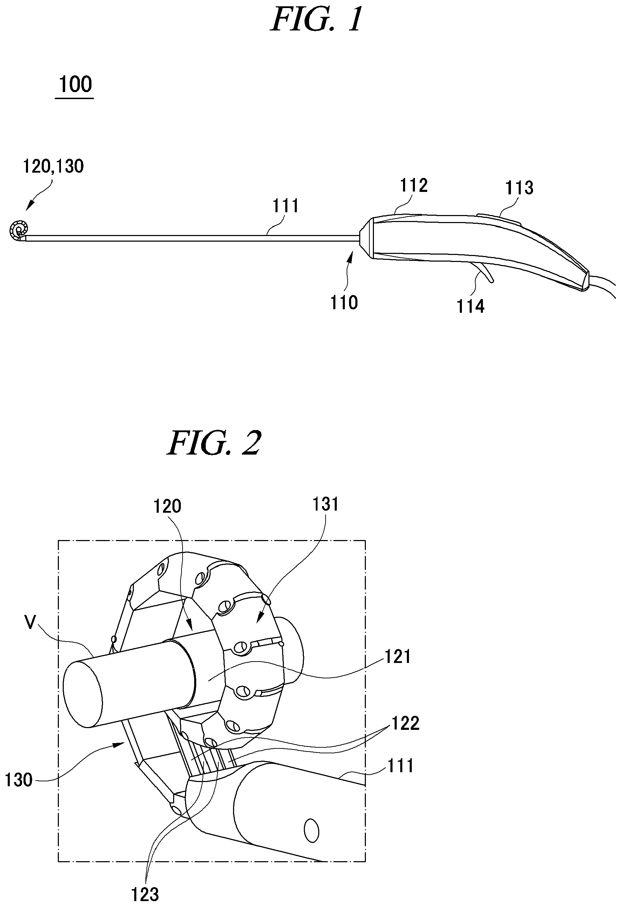

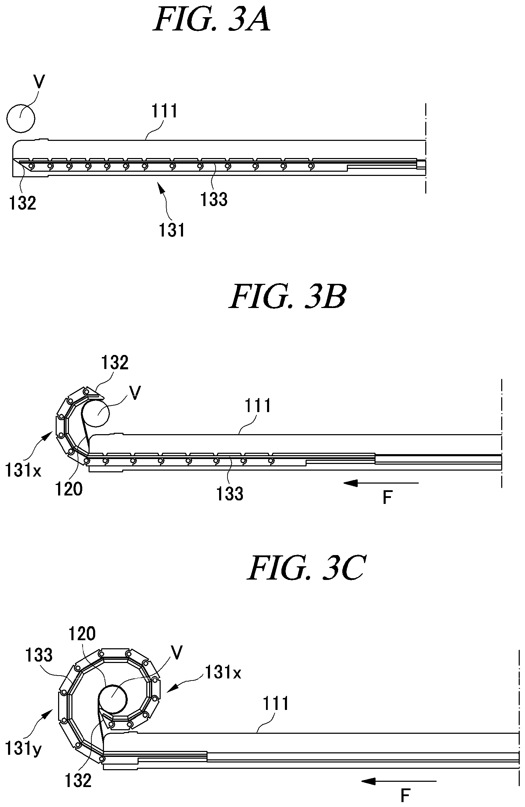

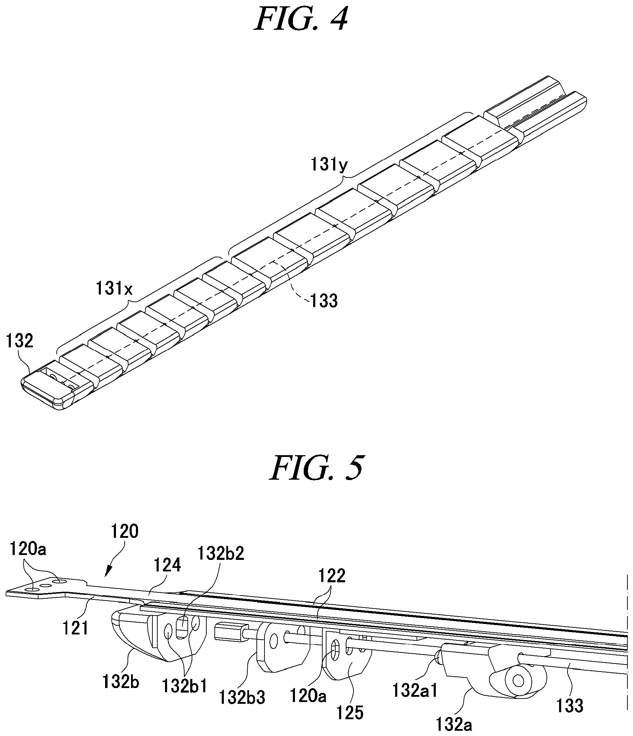

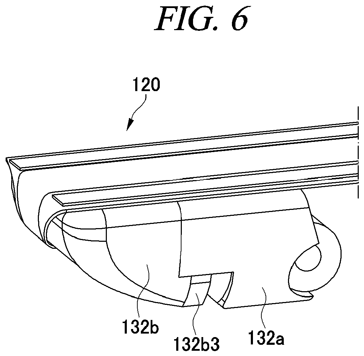

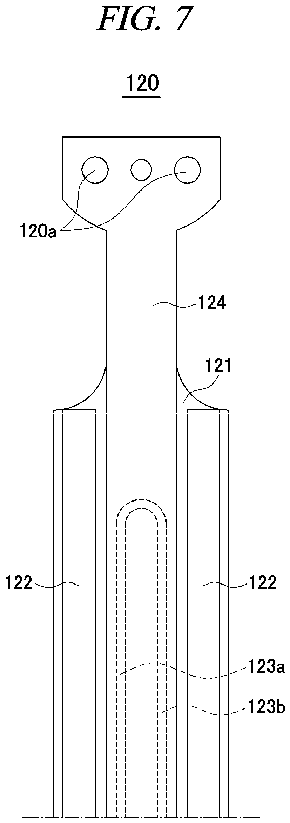

**FIG. 1** is a side view of an electrode apparatus according to an embodiment of the present disclosure. **FIG. 2** is a perspective view illustrating a wound state of an electrode guide illustrated in **FIG. 1****.** **FIG. 3A** through **FIG. 3C** illustrate a process of deforming the electrode guide into a wound state according to an embodiment of the present disclosure. **FIG. 4** is a perspective view illustrating a body portion and a coupling portion illustrated in **FIG. 2****.** **FIG. 5** is an exploded perspective view illustrating an electrode unit of the coupling portion illustrated in **FIG. 4****.** **FIG. 6** is a perspective view illustrating a state in which the electrode unit and the coupling portion are coupled to each other illustrated in **FIG. 5****.** **FIG. 7** is a plan view illustrating a portion of the electrode unit illustrated in **FIG. 5****.**

Referring to **FIG. 1****,** an electrode apparatus 100 according to an embodiment of the present disclosure includes a main body 110, an electrode unit 120 and an electrode guide 130. The main body 110 may include the shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate an operation of the electrode unit 120. The components for driving and controlling the electrode unit 120 and the electrode guide 130 may be located inside the main body 110.

The electrode unit 120 is formed to be drawn out from one end of the shaft 111 and configured to denervate or modulate at least part of nerves distributed on a tissue V in the body including a tube depending on manipulation by the operator. For example, the electrode unit 120 may be brought into contact with a tube-shaped tissue V in the body by enclosing an outer surface of the tissue V and may transmit electrical impulses or various energies to the nerves distributed on the tissue V to denervate or modulate at least some of the nerves.

Referring to **FIG. 2****,** the electrode unit 120 may include a base layer 121, an electrode layer 122 and a sensor unit 123. In the electrode apparatus 100 according to the present disclosure, an electrode encloses an outer surface of a tube or tube-shaped tissue V in the body and energy can be transferred through the electrode. To this end, the electrode unit 120 may be formed as a flexible printed circuit board (FPCB).

The electrode layer 122 is formed on the base layer 121, and in the embodiment illustrated in **FIG. 2****,** the electrode layer 122 may be composed of two electrodes extending parallel to each other on the base layer 121. In the present embodiment, the base layer 121 and the electrode layer 122 may be configured to extend in a circumferential direction and enclose the tube V in the body or the like.

The electrode layer 122 may be made of a material such as stainless steel or gold, which is harmless to the human body and conducts electricity well, in order to block or denervate or control or modulate the nerves. Also, the electrode layer 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Also, the electrode unit 120 may be implemented as a flexible PCB for transferring RF energy, a transducer for transferring ultrasonic energy or a metal electrode for transferring high-voltage energy and thus may transfer energy to damage the nerves.

Further, the sensor unit 123 may be formed on the base layer 121. For example, the sensor unit 123 may be a thermocouple that measures a temperature by contacting with the tube V in the body or the like, and when neurotomy is performed with the electrode apparatus 100 according to the present disclosure, the sensor unit 123 may monitor a temperature of a treatment site. As another example, the sensor unit 123 may measure signals from the nerves on the tube.

The electrode guide 130 functions to bring the electrode unit 120 into contact with the tube V in the body. The electrode guide 130 is coupled to the electrode unit 120 and deformed into a wound state to bring the electrode unit 120 into contact with the tube V in the body.

Referring to **FIG. 3A** through **FIG. 3C** and **FIG. 4****,** the electrode guide 130 of the present disclosure includes a body portion 131 and a coupling portion in order to be deformed into the wound state. In the wound state, the plurality of joint units 131 is disposed to enclose the circumference of the tube V in the body with the electrode unit 120 interposed therebetween. For example, the state illustrated in **FIG. 2** and **FIG. 3C** may be the wound state.

According to an embodiment of the present disclosure, the electrode guide 130 is accommodated together with the electrode unit 120 inside the shaft 111 and may protrude from one end in a forward direction F while being deformed into the wound state at the time of surgical procedure. As illustrated in **FIG. 3A** through **FIG. 3C****,** when the body portion 131 is sequentially drawn out, the body portion 131 may move toward one direction and thus may overall enclose the tube V in the wound state. In the wound state, the electrode guide 130 is spaced apart from an outer circumferential surface of the tube and the electrode unit 120 located inside the wound electrode guide 130 may be in close contact with the outer circumferential surface of the tube V.

According to an embodiment of the present disclosure, the body portion 131 may include a first body group 131x and a second body group 131y. As illustrated **in** **FIG. 3A** through **FIG. 4****,** the first body group 131x may have a first length in a longitudinal direction, and the second body group 131y may have a second length greater than the first length. According to the embodiment of the present disclosure, each of the first body group 131x and the second body group 131y may include, for example, six joints respectively having different lengths.

Due to such a difference in length, the first body group 131x may form a first radius of curvature and the second body group 131y may form a second radius of curvature greater than the first radius of curvature in the wound state. As can be seen from **FIG. 3C****,** the joint units (the first body group 131x) having a relatively small length may form a smaller radius of curvature and the joint units (the second body group 131y) having a relatively great length may form a greater radius of curvature.

More specifically, the first body group 131x forming the first radius of curvature may be located close to the coupling portion 132, and the second body group 131y forming the second radius of curvature may be located close to the shaft 111.

When the body portion 131 located close to the coupling portion 132 form a smaller radius of curvature in the wound state, a path along which the coupling portion 132 enters a space between the tube in the body and the shaft 111 may be formed as shown in **FIG. 3C****.** For example, the electrode guide 130 including the body portion 131 may have an overall spiral shape.

Hereafter, the coupling portion 132 will be described with reference to **FIG. 5** and **FIG. 6****.** The coupling portion 132 is connected to the end of the body portion 131 and configured to fix an end of the electrode unit 120. The coupling portion 132 may include a first clamping piece 132a and a second clamping piece 132b to fix or support the end of the electrode unit 120. As illustrated in **FIG. 5****,** the first clamping piece 132a may be fixed to the body portion 131, and the second clamping piece 132b may be coupled to the first clamping piece 132a with the first clamping piece 132a and a portion of the electrode unit 120 interposed therebetween. For example, the first clamping piece 132a may be disposed close to the body portion 131 of the electrode guide 130 and the second clamping piece 132b may be disposed close to the electrode unit 120.

Hereafter, a coupling structure of the first clamping piece 132a and the second clamping piece 132b will be described. Any one of the first clamping piece 132a and the second clamping piece 132b according to an embodiment of the present disclosure may include a protrusion 132a1 protruding in one direction and the other one of the first clamping piece 132a and the second clamping piece 132b may include a fastening groove 132b1 recessed corresponding to the protrusion 132a1. For example, if the protrusion 132a1 is provided in the first clamping piece 132a, the fastening groove 132b1 may be provided in the second clamping piece 132b, or if the protrusion is provided in the second clamping piece 132b, the fastening groove may be provided in the second clamping piece 132b as shown in **FIG. 5****.**

The protrusion 132a1 provided in the first clamping piece 132a may penetrate the electrode unit 120 and may be coupled and fastened to the fastening groove 132b1 provided in the second clamping piece 132b.

Also, a through-hole 120a through which the protrusion 132a1 penetrates may be formed at a portion of the electrode unit 120. Referring to **FIG. 5****,** the through-hole 120a may be formed at the end of the electrode unit 120 and may be formed between the first clamping piece 132a and the second clamping piece 132b. For example, the through-hole 120a formed at a portion of the electrode unit 120 may allow penetration of the protrusion 132a1 provided in the first clamping piece 132a.

The electrode unit 120 according to an embodiment of the present disclosure may extend to enclose the second clamping piece 132b and may be inserted between the first clamping piece 132a and the second clamping piece 132b.

Referring to **FIG. 3A** through **FIG. 5****,** the electrode guide 130 according to the present disclosure, the electrode guide 130 may further include a joint wire 133 which penetrates the body portion 131 and is coupled to the second clamping piece 132b to guide the body portion 131 to be in the wound state.

For example, with further reference to **FIG. 5****,** the joint wire 133 may be formed to sequentially penetrate the body portion 131. The joint wire 133 can guide the electrode guide 130 to be deformed into a shape enclosing the tube V in the body.

Referring to **FIG. 5****,** the second clamping piece 132b may include a wire groove 132b2 formed to allow insertion of an end of the joint wire 133 and a fixing plate 132b3 configured to close a portion of the wire groove in order to suppress deviation of an end of the joint wire 133 from the wire groove 132b2.

For example, the wire groove 132b2 may be formed to accommodate an end of the joint wire 133 between a plurality of fastening grooves 132b1 provided in the second clamping piece 132b. Referring to **FIG.** 5 and **FIG. 6****,** the end of the joint wire 133 inserted into the wire groove 132b2 may have a greater diameter than the wire 133 or may have a different shape from the wire 133 and thus may be caught and fixed in the fixing plate 132b3 without deviation from the wire groove 132b2.

Referring to **FIG. 7****,** the electrode unit 120 according to an embodiment of the present disclosure may include the base layer 121, the electrode layer 122 and a top layer 124. In the embodiment illustrated in **FIG. 7****,** the electrode unit 120 may include the base layer 121, the electrode layer 122 disposed on the base layer 121 and the top layer 124 disposed to overlap a portion of the electrode layer 122 with the electrode layer 122 interposed therebetween. Meanwhile, the sensor unit 123 may be a thermocouple composed of a first metal 123a and a second metal 123b, for example, a pair of copper and constantan.

The electrode unit 120 according to an embodiment of the present disclosure may further include a reinforcing layer 125 of which a part is coupled to the base layer 121 and the other part is inserted and fixed in the coupling portion 132. In the embodiment illustrated in **FIG. 5****,** the reinforcing layer 125 may also include the through-hole 120a.

At least one of the base layer 121 and the top layer 124 may be extended to enclose a part of the coupling portion 132 (for example, the second clamping piece 132b) and may be inserted and fixed in the coupling portion 132 in an opposite direction (from bottom to top in **FIG. 5** and **FIG. 6****)** to a direction (from top to bottom in **FIG. 5** and **FIG. 6****)** of insertion of the other part of the coupling portion 132. Thus, the electrode unit 120 may be coupled to enclose a part of the coupling portion 132 (for example, the second clamping piece 132b) in either direction.

According to the electrode apparatus 100 of the present disclosure, the electrode guide 130 and the electrode unit 120 may be spaced apart from each other in most sections except the coupling portion 132 in the wound state where they are located for surgical procedure. That is, the separation space between the electrode unit 120 and the electrode guide 130 is exposed to an environment such as air, and, thus, heat generated from the electrode unit 120 during energy transfer can be easily dissipated through convective heat transfer.

Also, according to the electrode apparatus 100 of the present disclosure, while a coupling (assembly) point between the electrode unit 120 and the electrode guide 130 is minimized, the electrode unit 120 can be stably supported by the coupling portion 132 of the electrode guide 130. Thus, durability and reliability of the apparatus can be secured. Further, the electrode unit 120 and the electrode guide 130 can be easily assembled with each other.

Furthermore, since the electrode guide 130 deformed into the wound state by power and the electrode unit 120 in close contact with the tube V in the body are configured separately from each other, it is possible to suppress the effects of power transmission, for example, transmission of physical energy such as a vibration or force to the tube in the body.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure. For example, each component described as a single type may be implemented in a dispersed form, and likewise components described as distributed may also be implemented in a combined form.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. An electrode apparatus for nerve denervation or modulation in body, comprising:
a main body including a shaft;
an electrode unit formed to be drawn out from one end of the shaft and configured to denervate or modulate at least some of nerves on a tube in the body; and
an electrode guide deformed into a wound state to bring the electrode unit into contact with the tube in the body,
wherein the electrode guide includes:
a body portion which is spaced apart from the electrode unit to enclose the circumference of the tube in the wound state; and
a coupling portion which is connected to the end of the body portion and to which an end of the electrode unit is fixed.

2. The electrode apparatus of Claim 1,
wherein the coupling portion includes:
a first clamping piece fixed to the body portion; and
a second clamping piece coupled to the first clamping piece with a portion of the electrode unit interposed therebetween.

3. The electrode apparatus of Claim 2,
wherein any one of the first clamping piece and the second clamping piece includes a protrusion protruding in one direction,
the other one of the first clamping piece and the second clamping piece includes a fastening groove recessed corresponding to the protrusion, and
a through-hole through which the protrusion penetrates is formed at a portion of the electrode unit.

4. The electrode apparatus of Claim 2,
wherein the electrode unit is extended to enclose the second clamping piece and inserted between the first clamping piece and the second clamping piece.

5. The electrode apparatus of Claim 2,
wherein the electrode guide further includes a joint wire which penetrates the body portion and is coupled to the second clamping piece to guide the body portion to be in the wound state, and
the second clamping piece includes:
a wire groove formed to allow insertion of an end of the joint wire; and
a fixing plate configured to close a portion of the wire groove in order to suppress deviation of the end of the joint wire from the wire groove.

6. The electrode apparatus of Claim 1,
wherein the electrode unit includes:
a base layer;
an electrode layer disposed on the base layer; and
a top layer disposed to overlap a portion of the electrode layer with the electrode layer interposed therebetween.

7. The electrode apparatus of Claim 6,
wherein the coupling portion includes:
a first clamping piece fixed to the body portion; and
a second clamping piece coupled to the first clamping piece with a portion of at least one of the base layer and the top layer interposed therebetween.

8. The electrode apparatus of Claim 7,
wherein any one of the first clamping piece and the second clamping piece includes a protrusion protruding in one direction,
the other one of the first clamping piece and the second clamping piece includes a fastening groove recessed corresponding to the protrusion, and
at least one of the base layer and the top layer includes a through-hole through which the protrusion penetrates.

9. The electrode apparatus of Claim 6,
wherein at least one of the base layer and the top layer is extended to enclose a part of the coupling portion and inserted and fixed in the coupling portion.

10. The electrode apparatus of Claim 6,
wherein the electrode unit further includes a reinforcing layer of which a part is coupled to the base layer and the other part is inserted and fixed in the coupling portion.

11. The electrode apparatus of Claim 10,
wherein at least one of the base layer and the top layer is extended to enclose a part of the coupling portion and is inserted and fixed in the coupling portion in an opposite direction to a direction of insertion of the other part of the coupling portion.

12. The electrode apparatus of Claim 6,
wherein the electrode unit further includes a sensor unit disposed on the base layer and configured to sense a temperature.
